Europäisches Patentamt

**European Patent Office**

**Office européen des brevets**

(19)

(11) Publication number:

**0 302 154**
**A1**

# EUROPEAN PATENT APPLICATION

(21) Application number: **87420346.6**

(22) Date of filing: **18.12.87**

(51) Int. Cl.⁴: **G01N 33/569**

(30) Priority: **20.07.87 US 75760**

(43) Date of publication of application:
**08.02.89 Bulletin 89/06**

(84) Designated Contracting States:
**AT BE CH DE ES FR GB GR IT LI LU NL SE**

(71) Applicant: SOFICORP SCIENTIFIC INC.
630 Dorchester Boulevard West
CDN-Montreal Quebec, H3B 1W2(CA)

(72) Inventor: Pekovic, Drasko D.
46 Place Kieffer
Dollard-des-Ormeaux Quebec H9B 1H8(CA)

(74) Representative: Laurent, Michel et al
Cabinet LAURENT et GUERRE 20, rue Louis
Chirpaz B.P. 32
F-69131 Ecully Cédex(FR)

(54) **Detection of HIV and anti-lymphocyte antibodies.**

(57) This invention relates to the detection of anti-bodies against HIV and anti-lymphocyte antibodies in sera of individuals previously exposed to HIV which react specifically with frozen section of HIV particles and normal blood lymphocytes. It is shown that the infection of human cells by HIV results in the production of antibodies which react specifically with HIV-core and the envelope thereof as well as with the surface and the intracellular structure of normal human blood lymphocytes. The presence, in biological specimens, of antibodies to the antigenic determinants localized on the HIV or blood lymphocytes is indicative of previous or current infection of an individual by HIV. Specifically, frozen sections of HIV and of the blood lymphocytes are specifically labelled by sera taken from individuals previously exposed to HIV. The preferred method of said detection of antibodies is by indirect immunofluorescence or by immunoperoxidase or by immunogold label-ling.

Fig. 1

EP 0 302 154 A1

## "DETECTION OF HIV AND ANTI-LYMPHOCYTE ANTIBODIES"

### BACKGROUND OF THE INVENTION

#### (a) Field of the Invention

The present invention relates to the immunochemical detection of antibodies against HIV and anti-lymphocyte antibodies in sera of individuals previously exposed to HIV, using sections of the viral particles and blood lymphocytes as antigens.

More specifically, this invention relates to the detection of antibodies against HIV and allogenic blood lymphocytes in sera of individuals exposed to the virus, using frozen sections and HIV and appropriate phenotype populations of blood lymphocytes from normal healthy donor as targets antigens. The invention intends to show that antibodies produced by the host immune system, after exposure to HIV reacts specifically with the virus and with HIV-uninfected normal blood lymphocytes. According to the invention, the preferred methods of detection include indirect immunofluorescence, immunoperoxidase and immunogold labelling.

#### (b) Description of Prior Art

1. Lapointe N. et al N. Engl. J. Med. vol. 312 pp. 1325-1326.
2. Pekovic D.D. et al. J. Virol. Meth. vol. 13 pp. 265-269, 1986.
3. Pekovic D.D. et al. Arch. Virol. vol. 91 pp. 11-19, 1986.
4. Pekovic D.D. et al. J. Pathol. vol. 152 pp. 31-35, 1987.
5. Pekovic D.D. et al. Amer. J. Med. vol. 82 pp. 188-189, 1987.
6. Robert C. Gallo et al, U.S. 4,520,113, May 28, 1985.

The acquired immune deficiency syndrome (AIDS) is the most severe immune system abnormality that has been linked to infection with the human immunodeficiency virus (HIV). The virus infects preferentially peripheral blood helper-inducer T cells phenotipically defined by the OKT4 marker. HIV selectively attaches to the receptor on these cells at a superficial locus that is either part or closely associated with the OKT4 complex of molecules. Following the attachment, the virus enters the cell and induces a suppression of the cell growth or a syncytium formation or immunopathologic reactions that lead to the eventual lysis of the cell. Given the importance of the helper-inducer cells (OKT4+) in the orchestration of the immune system, it becomes clear why their depletion is followed by the rapid progression of immuno-suppression and by the enhanced frequency of opportunistic infections and neoplasms.

The scope of the immune response to HIV infection has not been fully delineated. However, specific HIV antibodies have been demonstrated in the sera of individuals infected with HIV. In addition to the HIV antibodies, it has been found that infected individuals possess serum antibodies that can effectively mediate HLA- and sex- antigen-independent lysis of autogenous uninfected lymphocytes and lymphocytes from healthy subjects. It is believed that these lymphocytolytic auto-antibodies, present in sera from the early stages of HIV infection, may be the major factor in the rapidly progressing immunosuppression and destruction of lymphocytes. This consideration is supported by the fact that a relatively low percentage of infected blood lymphocytes cannot explain the rapid progression of the generalized lymphopenia.

Furthermore, individuals in high risk groups display in their serum a polyclonal gammopathy indicative of a state of B-lymphocyte disregulation. Many of these individuals have been infected with the virus that causes infectious mononucleosis which is known to induce this type of response. To characterize these individuals as immune-deficient is too strong a statement, but their immune systems are already undergoing abnormal activation. In the case of drug abusers, and perhaps even in the case of selected homosexuals, there is the possibility that drugs, toxins, or other unknown agents contribute to a subliminal state of immune deficiency, characterized by polyclonal gammopathy and production of anti-lymphocyte auto-antibodies. If that should be the case, their infection with HIV would only serve to magnify the subsequent immune deficiencies, and production of anti-lymphocyte auto-antibodies.

Most of the existing commerical kits consist of purified viral antigens, from lysate or purified proteins of HIV, which serve as targets for the detection of specific antibodies present in body fluids in HIV-positive individuals. This production entails complex biochemical procedures which may change the antigenic properties of those viral structures.

The present invention offers possibilities for the preservation of the viral antigens in their native form. Antibodies present in body fluids of pre-AIDS and AIDS individuals react readily and specifically with all viral structures in section of viral particle.

The present invention would permit new immunodeficiency viruses to be incorporated as target antigens as well.

It is an object of the present invention to provide a sensitive method for the detection of previous exposure to HIV by demonstrating the presence of HIV antibodies in body fluids of exposed individuals.

It is another object of the present invention to provide a technology for the detection of viral antibodies in sections of infected cells.

It is another object of the present invention to provide method for the detection of anti-lymphocyte antibodies in body fluids.

A further object of the present invention is to provide an efficient method for the detection of antibodies against HIV and/or anti-lymphocyte antibodies in body fluids of AIDS and pre-AIDS individuals.

It is another object of the present invention to provide a novel method for the detection of HIV antibodies in body fluids of HIV positive individuals.

It is another object of the present invention to provide a novel method for the detection of anti-lymphocyte antibodies in body fluid of people at risk for AIDS and HIV-positive individuals.

It is a still further object of the present invention to provide a method for identifying HIV-positive individuals who may elude detection by other tests.

It is another object of the present invention to provide a technology for the detection of antibodies to other human or animal viruses.

It is another object of the present invention to provide a technology for the detection of anti-lymphocyte antibodies which are present in body fluids of individuals with auto-immune abnormalities.

Another object of the present invention is to provide a test kit comprising sections of HIV pellet and blood lymphocytes, detection substrates and reagents necessary to carry out the test.

It is an object of the present invention to provide a novel method for the detection of anti-lymphocyte auto-antibodies in high risk individuals and to readily and easily detect these antibodies in sera or other body fluids of HIV positive patients.

It is another object of the present invention to show that the specificity of the antibodies with regard to ultrastructure of phenotypic target cells intensifies and that the pattern of labelling changes with the evolution of AIDS.

It is another object of the present invention to provide a complementary analysis of great diagnostic and prognostic potential by monitoring both antibodies against HIV in individuals previously exposed to HIV and anti-lymphocyte antibodies in individuals at risk to contract AIDS and HIV infected patients.

It is another object of the present invention to maximize the sensitivity of detection of these antibodies by using sections, preferably frozen, allowing maximal preservation of all viral and lymphocyte epitopes rather than selected, chemically purified antigens.

It is another object of the present invention to demonstrate the specificity of these antibodies by localizing the reaction with the ultrastructure in sections of target antigens.

## SUMMARY OF THE INVENTION

The invention relates to a method for the detection of antibodies which specifically bind to ultrastructures of HIV and/or of normal blood lymphocytes. The method consists of using samples of body fluids of individuals previously exposed to HIV, and sections of viral particles of HIV and/or sections of normal blood lymphocytes. The sections and serum or body fluids samples are brought into contact and formation of an antigen-antibody complex is allowed to take place. Any formation of the complex is subsequently detected by an immunoassay.

## DESCRIPTION OF PREFERRED EMBODIMENT

HIV is collected from a supernatant of HIV-infected culture of peripheral blood lymphocytes, embedded in 1% agarose, frozen in liquid nitrogen and sections produced using cryo-ultramicrotome. $OKT4^+$, $OKT8^+$, B-cells and monocytes, are separated from blood by fluorescence activated cell sorter (FACS), pellets of cells embedded in agarose, frozen in liquid nitrogen and sectioned using a cryo-ultra-microtome.

Sections of $OKT4^+$ cells, $OKT8^+$, B-cells and monocytes may be reacted with sera of homosexual males, drug addicts, and sera from individuals suspected to be infected by HIV. Indirect immunofluorescence immunoperoxidase and immunogold assays demonstrate whether the sera contain HIV Ab reacting with sections of the viruses and/or sections of phenotype population of blood lymphocytes.

## GENERAL DESCRIPTION

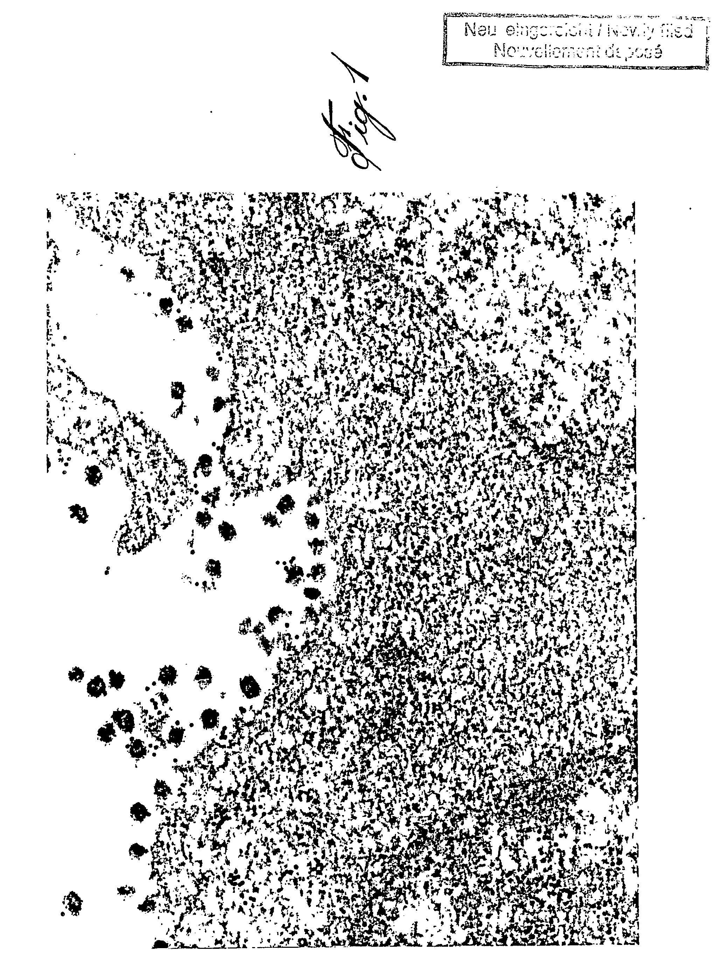

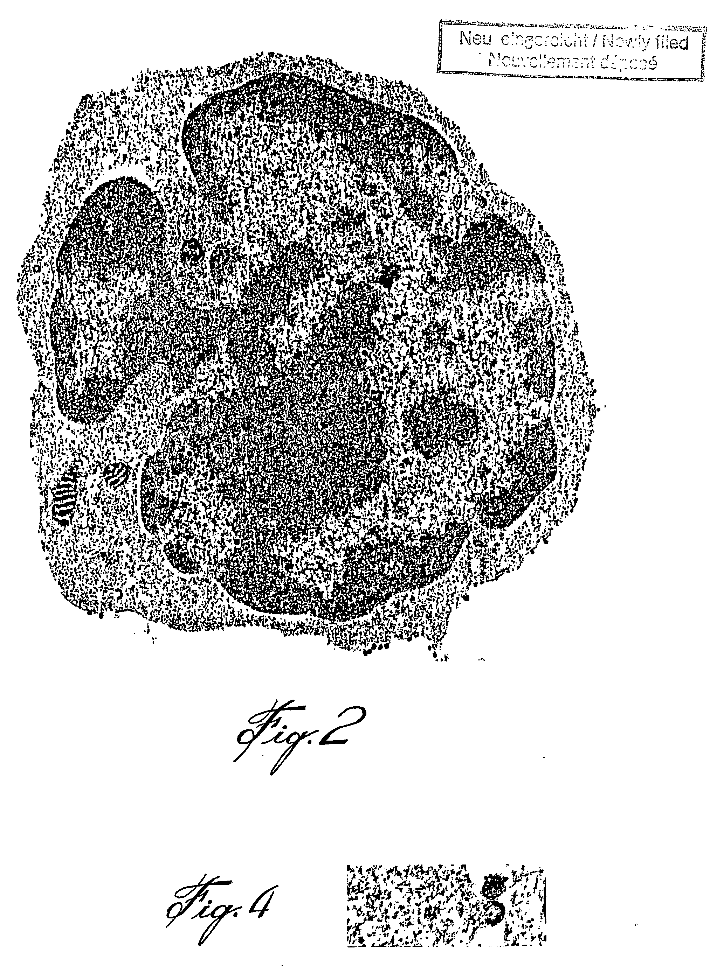

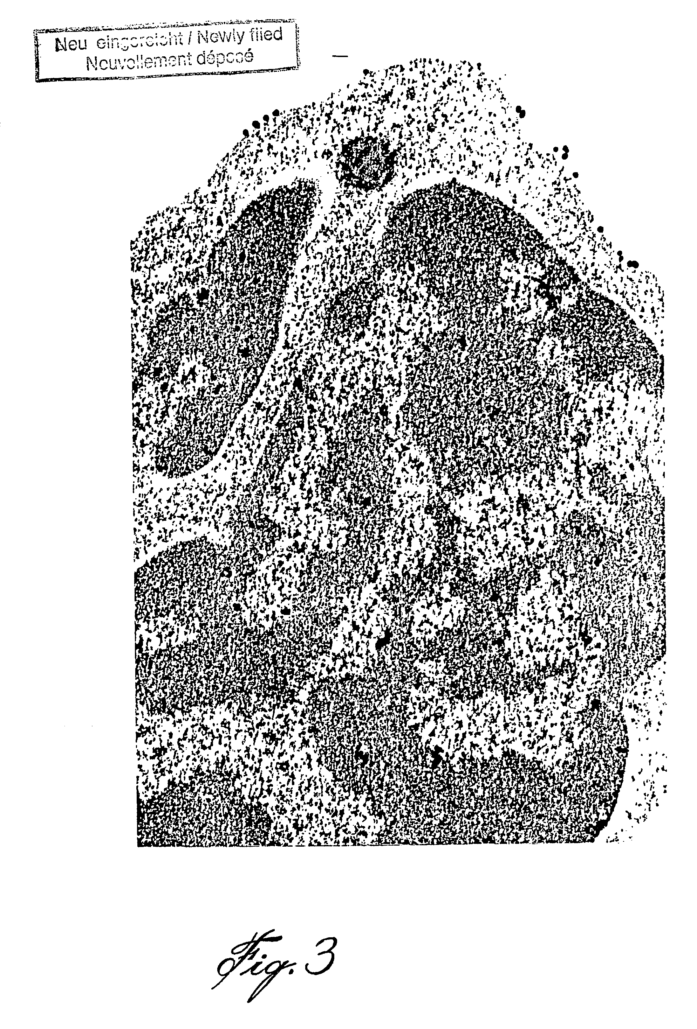

Frozen sections of pellets of viral particles of HIV; HIV-infected H9 cells, as well as frozen sections from normal $T4^+$, $T8^+$, B-cells and monocytes are tested with human sera using immunofluorescence and immunoelectron microscopy. The sera used for the analysis are also tested by Western blot technique. Sera from some healthy homosexual males and drug addicts as well as sera from HIV-infected individuals at different stages of evolution of the disease recognize directly core and envelope antigens in sections of viral particles and ultrastructures of HIV-infected H9 cells not detectable by any other means. Antibodies reacting with HIV in sections of HIV-infected H9 cells react mostly with the core of viral particles. Much stronger reactions is observed with the cell ultrastructure, as illustrated by Figure 1. Similarly, sera of some healthy homosexual males, drug addicts and all sera of individuals with clinical findings suggestive for AIDS strongly react with sections of all blood lymphocytes. However, the intensity of the reaction is different among sera from different categories of individuals. The pattern and intensity of the reaction of a single serum may be different among phenotypic lymphocyte population as illustrated in Figures 2 and 3. The pattern and intensity of the reaction changes with the progression of AIDS.

DETECTION OF ANTIBODIES AGAINST HIV

HIV is concentrated by ultracentrifugation from a virus producer culture supernatant (normal peripheral blood lymphocytes/HIV or permissive cell line/HIV). After removal of cell debris and non viral products by centrifugation through a cushion of 20% (W/W) sucrose in TNE buffer (10mM Tris-NaCl, pH 7.4, 0.1M NaCl and 0.001 mM EDTA) the virus particles are purified by equilibrium density banding through a linear gradient of 20-60% sucrose (W/W) in TNE. The gradient is divided into several fractions and the virus band located by assaying aliquots of each fraction for HIV specific labelling by immunoelectron microscopy.

A pellet of the viral particles 1 x 10$^8$ prt 0.1 ml is fixed, embedded in 0.1 ml of 1% agarose without electrical charge, frozen in liquid nitrogen and sectioned at 700-900A using a cryo-ultramicrotome. The immunofluorescence for the detection of anti-HIV antibodies in patient sera includes indirect labelling. Secondary antibodies or specific F(ab')2 fragments can be conjugated by fluorochromes, peroxidase or colloidal gold particles of optical microscopy grade.

Antibodies reacting with frozen sections of HIV are detected in sera of patients with AIDS and immunodeficiency conditions preceding AIDS by the process of the present invention. Example 1 is a detailed description of the present invention using the indirect IF technique.

The immunofluorescence technique involves reacting a section of a pellet of HIV particles with the test sera. The immune complexes produced are labelled by fluorochrome- conjugated anti-human immunoglobulin antibodies or F(ab)2 framents of IgG. Any section indicating the presence of HIV antibodies demonstrates a detectable and measurable colored product. Ultrathin sections mounted on 400-mesh naked-nickel grids are incubated with the test sera give rise to the immune complexes labelled with colloidal gold complex. This technique allows for the detection of antibodies reacted with the HIV envelope or core or both as illustrated in Figure 4.

DETECTION OF ANTI-LYMPHOCYTE ANTIBODIES

Peripheral blood lymphocytes are separated on a Ficoll-hypaque gradient from the blood of a healthy individual. Sex and HLA antigens are blocked by incubation with respective specific commercial sera and Fc receptors were blocked by incubation with normal human immunoglobulins. Phenotype populations of OKT4$^+$, OKT8$^+$ and B lymphocytes and from monocytes are separated by FACS. Pellets containing 2 x 10$^6$ of cells of each cell phenotype are embedded in 0.2 ml of 1% agarose, frozen in liquid nitrogen and sectioned at 1-2 um using a cryostat or at 700-900A using a cryo-ultramicrotome. The sections are mounted on slides for optical and ultraviolet microscopy.

Antibodies to normal lymphocytes are detected in sera of patients suspected to have previously been exposed to HIV, by the process of present invention. Anti-lymphocyte antibodies are detected by indirect immunofluorescence. Example 1 is a detailed description of the present invention using immunofluorescence technique. Also, a usable and most precise technique for the detection of anti-lymphocyte antibodies, is the post-embedding immunoelectron microscopy.

The immunofluorescence technique for the detection of anti-lymphocyte antibodies consists of reacting the section of pellets of purified phenotype populations of blood lymphocytes with sera obtained from blood of patients suspected to have previously been exposed to HIV. The reaction is visualized by labelling of the reacted patient antibodies with fluorochrome conjugated commercial antibodies or F(ab')2 fragments of IgG against human immunoglobulins. Sections positive for anti-lymphocyte antibodies form a detectable and measurable colored product. As indicated above antibodies to normal lymphocytes may also be detected in sera of patients suspected to have been exposed to HIV by means of immunoelectron microscopy. Ultra-thin sections of pellet of phenotype population of blood lymphocytes is incubated with sera of the patients and reaction labelled with commercial immunogold complex against human immunoglobulins. This technique allows the characterization of the specificity of antibodies according to the labelled cell ultrastructure.

BRIEF DESCRIPTION OF FIGURES

FIGURE 1 is an electronmicrograph showing HIV infected H9 cell labelled with chromatographically purified IgG against HIV obtained from serum of an AIDS patient and protein A-gold complex 15 nm in diameter. Figure 1 shows strong labelling of intracellular structures and with labelling of viral particles located on the surface of the cell. G 98500.

FIGURE 2 is an electronmicrograph showing the reaction of anti-lymphocyte serum antibodies from an adult AIDS patient with OKT4$^+$ lymphocyte from a normal subject. Double immunogold labelling. OKT4 marker was labelled prior to embedding of the cell with OKT4 monoclonal antibodies and goat anti-mouse IgG-gold complex 30 nm in diameter. Sections were incubated with electrophoretically purified IgG from an adult AIDS patient. The reaction of anti-lymphocyte Ab-section were labelled with protein A-gold complex 15 nm in diameter. G 56800.

FIGURE 3 is an electronmicrograph showing the reaction of anti-lymphocyte serum antibodies from a child with AIDS with OKT8$^+$ lymphocyte from a normal subject. Double immunogold labelling. OKT8 marker was labelled prior to embedding of the cell with OKT8 monoclonal antibodies and goat anti-mouse IgG-gold complex 30 nm in diameter. Sections were incubated with electrophoretically purified IgG from a child with AIDS. Reaction of anti-lymphocyte Ab-section were labelled with protein A-gold complex 15 nm in diameter.

FIGURE 4 is an electronmicrograph showing a section of budding and a mature viral particle of HIV labelled with electrophoretically purified IgG from serum from an adult patient with AIDS and protein A-gold complex 15 nm in diameter.

The invention is illustrated by means of the following examples.

Example 1

Immunofluorescence assay for the detection of HIV antibodies.
Indicator antigen - frozen section of HIV particles
Test serum - serum of individuals previously exposed to HIV
Negative controls - sections of other human T-cell lymphotropic retroviruses
- normal human serum.

HIV was concentrated by ultracentrifugation from supernatant of HIV-infected human periphenal blood lymphocytes. All debris and non viral products have been removed by centrifugation through a cushion of 20% (w/w) sucrose in TNE buffer (10 um TRIS-NaCl, pH 7.4, 0.1 M NaCl and 0.001 uM EDTA). HIV particles were purified by equilibrium density banding through a linear gradient of 20-60% sucrose (w/w) in TNE. The virus band was located by immunoelectron microscopy assay using P24 monoclonal antibodies. A pellet of viral particles was fixed, embedded in 1% agarose without electrical charge, frozen in liquid nitrogen and sectioned at 700-900A using a cryo-ultramicrotome. The sections were mounted on a slide and used for the detection of anti-HIV antibodies in sera of individuals previously exposed to HIV using indirect immunofluorescence assay.

Frozen sections were mounted 3 per slide, fixed by air drying, washed 3 times for 10 minutes in a phosphate buffer solution at pH 7.4. The sections were air-dried again and were post-fixed in acetone for 10 min. The middle section of each slide is used as control for the inhibition of fluorescence with the specific non-conjugated secondary antibodies. The remaining 2 sections on the slide were used for the demonstration of HIV fluorescence. The first incubation (30 min. at room temperature) with sera from individuals suspected to have been exposed to HIV allowed the reaction of serum antibodies with viral structures. The sections were washed 3 times and air dried as above. The second incubation (30 min. room temperature) with FITC-conjugated IgG against human immunoglobulins allowed labelling of complexes patient serum antibodies-virus. The sections are again washed 3 times and air-dried as above; mounted on glycerol buffered at pH 9.5 and examined on a Dialux epifluorescence microscope.

Example 2

Immunofluorescence assay for the detection of anti-lymphocyte antibodies.
Indicator cells - normal human lymphocytes
Test serum - serum of individuals previously exposed to HIV
Negative control - Normal human serum

Peripheral blood lymphocytes were separated on a Ficoll-hypaque gradient from the blood of a healthy subject, T4$^+$ and T8$^+$ lymphocytes, B-cells and monocytes were separated by FACS. Pellets containing $2 \times 10^6$ of cells of each phenotype population were embedded in 0.2 ml of one percent agarose, immediately frozen in liquid nitrogen and sectioned in 700-900A sections using a cryo-ultramicrotome. The sections were mounted on a slide and used for the detection of anti-lymphocyte antibodies in indirect immunofluorescence assay. The frozen sections were mounted 3 per slide and fixed by air-drying, then they were washed 3 times, 10 minutes in the phosphate buffer solution at pH 7.4. The sections were air-dried again, and were post-fixed in acetone for 10 min. The middle section on each slide is used as control for the inhibition of fluorescence with the specific, non-conjugated secondary antibodies. The remaining 2 sections on each slide were both used for the demonstration of fluorescence. The sections were first incubated with sera of HIV-positive patients and allowed to react for 30 minutes at room temperature, and washed 3 times as indicated above. The second incubation was carried out with FITC-conjugated F(ab')2 fragments of goat anti-human immunoglobulins, washed again 3 times, air-dried, mounted in glycerol buffered at pH 9.5 and examined on a Dialux™ epifluorescence microscope.

Finally, a different AIDS specific test kit for detecting antibodies against HIV and antibodies against lymphocytes using indirect immunofluorescence assay comprises a compartmented container, human serum containing HIV antibodies normal human serum, fluorochrome or peroxidase conjugated goat IgG against human IgG and IgM or protein A colloidal gold complex slides containing two rows of sections (one row comprises two sections of HIV and sections of antigenically similar or different viruses; the second row of sections contains the sections of T4⁺, T8⁺ and B lymphocytes and monocytes), and mounting medium.

## Claims

1. A method for the demonstration of antibodies which specifically bind to ultrastructures of HIV which comprises
providing samples of body fluids of individuals previously exposed to HIV, or fractions thereof,
providing sections of viral particles of HIV,
contacting said samples or fractions thereof with said sections and allowing formation of antigen-antibody complex to take place, and
detecting any formation of said antigen-antibody complex by an immunoassay.

2. A method according to claim 1, for detection of antibodies which additionally bind to normal blood lymphocytes, which comprises also providing sections of normal blood lymphocytes, and contacting said samples or fractions with both said sections.

3. A method for the detection of antibodies which specifically bind to normal blood lymphocytes which comprises
providing samples of body fluids of individuals previously exposed to HIV, or fractions thereof,
providing sections of normal blood lymphocytes,
contacting said samples or fractions thereof with said sections and allowing formation of antigen-antibody to take place, and
detecting any formation of antigen-antibody complex by immunoassay.

4. A method according to claim 1, wherein said immunoassay is selected from the group consisting of immunofluorescence, immunoperoxidase and immunogold labelling.

5. A method according to claim 1, wherein said samples or fractions thereof are taken from individuals at pre AIDS stage.

6. A method according to claim 1, wherein said samples are taken from individuals at the early stage of AIDS disease.

7. A test kit for detecting antibodies against HIV and antibodies against lymphocytes which comprises a compartmented container containing a human test serum containing HIV antibodies, a plurality of slides, each slide containing at least two rows of sections, said first row comprising sections of HIV and sections of antigenically similar or different viruses, said second row comprising sections of T4⁺, T8⁺, B lymphocytes and monocytes, a mounting medium and an agent selected from the group consisting of fluorochrome, peroxidase conjugated goat IgG against human IgG and IgM and protein A colloidal gold complex.

8. A method according to claim 3, wherein said immunoassay is selected from the group consisting of immunofluorescence, immunoperoxidase and immunogold labelling.

9. A method according to claim 3, wherein said samples or fractions thereof are taken from individuals at pre AIDS stage.

10. A method according to claim 3, wherein said samples are taken from individuals at the early stage of AIDS disease.

EP 0 302 154 A1

Fig. 1

Fig. 2

Fig. 4

Fig. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 196 873 (CENFOLD HOLDINGS S.A.) * Page 2, line 34 - page 3, line 7; page 4, lines 18-26; page 5, line 19 - page 6, line 14; page 12, lines 2-35 * | 1-10 | G 01 N 33/569 |
| X | WO-A-8 504 903 (UNITED STATES OF AMERICA DEPARTMENT OF COMMERCE & LITTON BIONETICS) * Page 3, line 20 - page 4, line 10; page 15, example 1; page 17, example 3; page 24, line 2 - page 30, line 5 * | 1-10 | |
| X | NATURE, vol. 327, 5th June 1987, pages 710-713, London, GB; R.B. STRICKER: "An aids-redlated cytotoxic autoantibody reacts with a specific angigen on stimulated CD4+T cells" * Page 710, column 1, lines 1-52 * | 2,3,5-7 ,8,10 | |
| X | FEDERATION PROCEEDINGS, Washington, DC, 29th March - 2nd April 1987, vol. 46, no. 4, 5th March 1987, page 1379, no. 6202; R.Q. WARREN et al.: "Two color fluorescence analysis of anti-lymphocytic autoantibodies in sera from homosexuals" * Whole abstract * | 2,3,5-7 ,8,10 | TECHNICAL FIELDS SEARCHED (Int. Cl.4) G 01 N |
| X | BRITISH JOURNAL OF HAEMATOLOGY, vol. 67, no. 1, 1987, pages 109-114, GB; J. VON DER LELIE et al.: "Autoimmunity against blood cells in human immunodeficiency-virus (HIV) infection" * Page 109, summary; page 109, column 2, line 14 - page 110, column 2, line 6 * | 1-3,5-7 ,8,10 | |

---      -/-

The present search report has been drawn up for all claims

| Place of search | Date of completion of the search | Examiner |
|---|---|---|
| THE HAGUE | 28-10-1988 | VAN BOHEMEN C.G. |

EPO FORM 1503 03.82 (P0401)

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | TRANSFUSION, vol. 25, no. 4, 1985, pages 308-312, US; E.G. SANDSTROM et al.: "Detection of human anti-HTLV-III antibodies by indirect immunofluorescence using fixed cells" * Page 308, abstract; page 308, column 2, line 16 - page 309, column 1, line 37 * | 1-10 | |
| Y | JOURNAL OF CLINICAL IMMUNOLOGY, vol. 7, no. 2, 1987, pages 130-139, Plenum Publishing Corp., US; G.E. OZTURK et al.: "The significance of antilymphocyte antibodies in patients with acquired immune deficiency syndrome (AIDS) and their sexual partners" * Page 130, column 1, line 1 - column 2, line 13; page 133, column 1, line 5 - column 2, line 5 * | 1-10 | |
| Y | EP-A-0 015 473 (F. HOFFMANN-LA ROCHE & CO.) * Page 2, lines 24-26; page 19, line 2 - page 20, line 19 * | 1-10 | **TECHNICAL FIELDS SEARCHED (Int. Cl.4)** |

The present search report has been drawn up for all claims

| Place of search | Date of completion of the search | Examiner |
|---|---|---|
| THE HAGUE | 28-10-1988 | VAN BOHEMEN C.G. |

EPO FORM 1503 03.82 (P0401)